# EUROPÄISCHE PATENTSCHRIFT

(11) **EP 0 526 472 B1**
(45) Veröffentlichungstag und Bekanntmachung des Hinweises auf die Patenterteilung: **12.04.1995**
(21) Anmeldenummer: 91906935.1
(22) Anmeldetag: 02.04.1991
(51) Int. Cl.: C08G 18/08, C08G 18/76, C09J 175/08

(54) **HAUSHALTSALLESKLEBER AUF POLYURETHANBASIS**
HOUSEHOLD GENERAL-PURPOSE ADHESIVE ON A POLYURETHANE BASIS
COLLE UNIVERSELLE A USAGE MENAGER A BASE DE POLYURETHANE

(30) Priorität: 09.04.1990 DE 4011455
(43) Veröffentlichungstag der Anmeldung: 10.02.1993
(73) Patentinhaber: Henkel Kommanditgesellschaft auf Aktien, 40191 Düsseldorf (DE)
(72) Erfinder: KLAUCK, Wolfgang, D-4005 Meerbusch 2 (DE); GIERENZ, Gerhard, D-5650 Solingen 19 (DE); MAIER, Wolfgang, D-4000 Düsseldorf 30 (DE); HÖFER, Rainer, D-4000 Düsseldorf 30 (DE); GRÜTZMACHER, Roland, D-5603 Wülfrath (DE)
(86) Internationale Anmeldenummer: EP9100630
(87) Internationale Veröffentlichungsnummer: WO9115529

(56) Entgegenhaltungen:
- EP-A- 0 197 170
- EP-A- 0 354 471
- EP-A- 0 369 389
- JOURANL OF CELLULAR PLASTICS, vol 18, no. 6, December 1982, Westport, Connecticut, US ; Arendt et al.:"M- and P-TMXDI : Two Isocyanates for the Polyurethane Industry ", pp. 373-383

## Beschreibung

Die Erfindung betrifft den Einsatz spezieller, im wesentlichen klarer, wäßriger und zumindest weitgehend lösemittelfreier Polyurethan-Dispersionen als Haushaltsalleskleber sowie ein Verfahren zu deren Herstellung.

Haushaltsalleskleber - auch Vielzweckkleber genannt - haben den Zweck, eine Vielzahl im Haushalt vorkommender Substrate (Papier, Pappe, Fotos, Textilien, Leder, Filz, Bast, Kork, Folien, Metalle, wie Aluminium und Eisen, Porzellan, Keramik, Glas, Holz, verschiedene Kunststoffe, wie auch z.B. Polystyrolschäume) zu verkleben. Dabei wird erwartet, daß auf dieser Vielzahl von Substraten, die sich in ihrer Oberflächenstruktur chemisch und physikalisch unterscheiden, und die üblicherweise vor dem Verkleben keiner speziellen Oberflächenbehandlung unterzogen werden, ausreichende Haftwirkung einstellt.

Im Vergleich zu der großen Vielzahl von Klebstoffklassen und -arten, die in Industrie und Handwerk eingesetzt werden, gibt es nur wenige Stoffe, die den hohen Anforderungen an die Universalität eines Haushaltsallesklebers gewachsen sind. Breite Verwendung hat dabei lediglich Polyvinylacetat und seine Copolymeren gefunden, das üblicherweise in Lösung oder für die Holzverklebung als Dispersion angeboten wird.

Die Forderung nach Universalität stellt für einen Klebstoff ein besonders hartes Auswahlkriterium dar. Sie bedeutet letztendlich, daß die Klebstoffmoleküle zu polaren wie unpolaren Grenzflächen hohe Affinität aufweisen müssen. Die Aussage, daß eine bestimmte Substanz als Klebstoff geeignet ist, gibt daher für den Fachmann noch keinen Hinweis, ob sie auch als Haushaltsalleskleber eingesetzt werden kann.

Neben der Forderung der Universalität besteht bei Haushaltsallesklebern neuerdings auch der Wunsch, geruchsneutrale, lösemittelfreie, physiologisch unbedenkliche, klare, wäßrige Formulierungen zu erreichen. Diese Formulierungen sollen jedoch gleichzeitig zu Klebstoffen führen, deren getrocknete Filme wiederum über eine gewisse Wasserfestigkeit verfügen. Darüber hinaus sollen diese wasserbasierten Klebstoffe auch in der Lage sein, schwierig klebbare Substrate, wie Kunststoffe, zu binden. Außerdem sollen sie eine hohe Lagerstabilität aufweisen.

Dieses Anforderungsprofil konnte weder auf Basis der bisher für Alleskleber bevorzugten Bindemittel Polyvinylacetat und Vinylacetat-Copolymere noch durch Alternativen, wie Nitrocellulose in allen Punkten erfüllt werden. Zwar kann Polyvinylacetat lösemittelfrei in Form wäßriger Dispersionen hergestellt werden, diese Dispersionen sind jedoch nicht transparent, sondern milchig-weiß. Sie zeigen z. B. gute Gebrauchseigenschaften bei Verwendung als Holzleim. Auch die als Dispersionskleber weit verbreiteten Acrylate und Styrol-Acrylate sind in Form transparenter Haushaltsklebstoffe mit den genannten Eigenschaften im Markt nicht bekannt.

Bekannt ist, daß einige speziell ausgewählte Ausführungsformen der seit Jahrzehnten bekannten wäßrigen Polyurethan-Dispersionen sich als Vielzweckalleskleber eignen. Polyurethan-Dispersionen bestehen aus Addukten mehrfunktioneller Isocyanate (Isocyanatkomponente) an mehrfunktionelle OH-Verbindungen (Polyolkomponente), die einkondensiert Bausteine enthalten, die in wäßriger Lösung zur Salzbildung befähigt sind. Gefunden wurde überraschenderweise, daß auch solche Polyurethan-Dispersionen als Haushaltsalleskleber geeignet sind und zu guten Haftungswerten führen, bei denen die zugrundeliegende Polyolkomponente auf Polypropylenglykol basiert und Tetramethylxyloldiisocyanat die Grundlage der Isocyanatkomponente bildet. Desweiteren wurde überraschenderweise gefunden, daß sich derartige Dispersionen ohne Verwendung inerter Lösungsmittel herstellen lassen.

In der deutschen Offenlegungsschrift DE 36 30 045 wird in breiter Weise ein Klebstoff auf Basis einer wäßrigen Dispersion von Polyurethanen mit eingebauten Carboxylat- und/oder Sulfatgruppen beschrieben, dessen zugrundeliegende Diisocyanatkomponente aus einem Gemisch mindestens zweier (cyclo)aliphatischer Diisocyanate besteht. Die Offenlegungsschrift nennt zahlreiche Polyole, zahlreiche Isocyanatverbindungen und zahlreiche salzbildende Komponenten als Modifizierungsmittel. Weiter genannt sind auch die hier üblichen Kettenverlängerungsmittel. Die Klebstoffe sollen zum Verkleben beliebiger Substrate geeignet sein.

Die hinsichtlich der Polyolkomponente breite Offenbarung der DE 36 30 045 umfaßt sowohl Polyurethan-Dispersionen, bei denen als OH-funktionelle Komponente Polyester eingesetzt werden, als auch solche, bei denen Polyether verwendet werden.

Polyurethan-Dispersionen auf Basis von Polyurethanen, die aus OH-funktionellen Polyestern aufgebaut sind, sind jedoch als Haushaltsalleskleber wenig geeignet, da sie bei Lagerung der Hydrolyse unterliegen und somit nicht die geforderte Lagerstabilität aufweisen.

Die DE 15 95 602 benennt zwar als mögliches Polyol beispielsweise auch Polymerisationsprodukte des Propylenoxids, es findet sich jedoch kein Hinweis darauf, daß derartige Polyole eine geeignete Basis für Polyurethan-Dispersionen für Haushaltsalleskleber mit dem genannten Anforderungsprofil darstellen.

Aus der Offenlegungsschrift DE 38 27378 sind zwar Haushaltsalleskleber mit dem genannten Anforderunsprofil auf Basis von Polyurethan-Dispersionen bereits bekannt, jedoch nicht solche Dispersionen, bei denen die Polyurethane durch Umsetzung von Polypropylenglykol mit Isocyanaten gebildet werden.

Zwar werden in der genannten DE 38 27 378 sowie in der korrespondierenden EP 354 471 unter vielen anderen Polyethern auch Polymerisationsprodukte des Propylenoxids genannt, jedoch nur als Beimischung zu der dort beschriebenen, aus Polytetrahydrofuran bestehenden Polyolmischung. Gleichzeitig wird in den beiden Schriften darauf verwiesen, daß Polyurethan-Dispersionen, denen als OH-funktionelle Komponente Polyethylenoxid und/oder Polypropylenoxid zugrundeliegen, ebenfalls als Vielzweckkleber nicht geeignet sind, da sie schlechte Haftung auf Kunststoffoberflächen zeigen und somit das Erfordernis der Universalität nicht erfüllen. Dies deckt sich mit der Aussage aus der DE-OS 17 69 387, daß Polyetherdiole, die aus Epoxiden wie Ethylenoxid oder Propylenoxid hergestellt worden sind, nicht als Polyolkomponente zur Herstellung von Polyurethandispersionen geeignet sind, die zum Verkleben von weichmacherhaltigen PVC-Kunststoffen eingesetzt werden sollen.

In der EP 354 471 wird neben vielen anderen geeigneten Isocyanaten auch Tetramethylxyloldiisocyanat genannt, jedoch findet sich kein Hinweis darauf, daß gerade die Kombination dieses Isocyanats mit einer auf Polypropylenglykol basierenden Polyolkomponente zu Polyurethan-Dispersionen führt, die bei der Verwendung als Vielzweckkleber doch gute Haftung z.B. auf Kunststoffoberflächen zeigt. Desweiteren findet sich kein Hinweis darauf, daß gerade bei dieser Kombination die Dispersion ohne Verwendung inerter Lösungsmittel hergestellt werden kann. Vielmehr wird in den beiden genannten Schriften das Acetonverfahren bevorzugt.

Aufgabe der Erfindung ist es daher, aufzuzeigen, daß spezielle wäßrige, transparente Polyurethan-Dispersionen auf Basis einer Polyolkomponente, die auf Polypropylenglykol beruht, den komplexen und teilweise widersprüchlichen vorher geschilderten Anforderungen an Haushaltsalleskleber genügen. Dazu gehört auch eine sehr gute Hydrolysestabilität und eine hohe Haftfestigkeit. Zudem soll ein Verfahren aufgezeigt werden, das es gegenüber dem bisherigen Stand der Technik ermöglicht, als Haushaltsalleskleber verwendbare Polyurethandispersionen herzustellen, ohne daß bei deren Herstellung inerte Lösungsmittel verwendet werden müssen, so daß die Dispersionen auch völlig frei von Restgehalten an Lösungsmitteln hergestellt werden können.

Gegenstand der Erfindung ist die Verwendung einer opaken bis wasserklaren und zumindest weitgehend lösemittelfreien wäßrigen einkomponentigen Polyurethan-Dispersion auf Basis der Reaktionsprodukte
- einer ganz oder teilweise aus Polypropylenglykol bestehenden Polyolmischung,
- einer ganz oder teilweise aus alpha-alpha-alpha'-alpha'-Tetramethylxyloldiisocyanat (TMXDI) bestehenden Mischung mehrfunktioneller Isocyanate,
- einer in wäßriger Lösung zur Salzbildung befähigten funktionellen Komponente und
- gewünschtenfalls eines Kettenverlängerungsmittels
als Haushaltsalleskleber.

Weiterer Gegenstand der Erfindung ist ein Verfahren zur Herstellung der als Haushaltsalleskleber verwendbaren Polyurethandispersionen.

Die den erfindungsgemäß eingesetzten Polyurethan-Dispersionen zugrundeliegenden Polyurethane basieren auf einer ganz oder teilweise aus Polypropylenglykol bestehenden Polyolmischung, wobei der Gehalt an Polypropylenglykol, bezogen auf Polyolmischungen, nicht kleiner als 30 und vorzugsweise nicht kleiner als 50 Gew.-% sein soll. Bevorzugt werden solche Mischungen, die über 70 Gew.-% Polypropylenglykol enthalten. Besonders geeignet ist eine gänzlich aus technischem Polypropylenglykol bestehende Polyolmischung. Diese Variante wird u.a. deshalb bevorzugt, weil einerseits daraus hergestellte Polyurethandispersionen bei anwendungstechnischen Tests gute Ergebnisse zeigen und andererseits das Herstellungsverfahren insofern vereinfacht wird, als daß ein Arbeitsschritt - das Zumischen einer oder mehrerer Polyole - entfällt. Auch isotaktisches Polypropylenglykol ist geeignet.

Dem Fachmann ist Polypropylenglykol als Verbindungsklasse gut bekannt, z.B. aus Kirk-Othmer, Encyclopedia of Chemical Technology, 3. Auflage, Band 18, John Wiley & Sons, New York 1982, S. 633-645. Polypropylenglykole sind theoretisch oder auch praktisch Addukte von Propylenoxid, gestartet auf einem Molekül mit mindestens einem aktiven Wasserstoff. Derartige Moleküle sind Alkohole, Polyalkohole, Amine oder auch wasser. Technisch bedeutsam sind Glycerin, Propylenglykol, Ethylenglykol und Trimethylolpropan. Von den Aminen sind beispielsweise Ethylendiamin, Diethylentriamin und Toluoldiamin geeignet. Weitere geeignete Startermoleküle als auch geeignete, kommerziell erhältliche Polypropylenglykole lassen sich ebenfalls der genannten Literaturstelle entnehmen. Als besonders günstig haben sich im Sinne der Erfindung Polypropylenglykole erwiesen, die einen Molekulargewichtsbereich von 200 bis 5.000, insbesondere 300 bis 3.000 aufweisen. Geeignete Polypropylenglykole können auch im Polymergerüst untergeordnete Mengen, insbesondere bis zu 10 Mol-% bezogen auf die Edukt-Monomeren, an anderen Alkyloxideinheiten, vorzugsweise Ethylenoxid, enthalten. Diese können im Polymeren statistisch verteilt und/oder blockweise enthalten sein.

Besonders vorteilhaft ist eine Ausführungsform der Erfindung, die es gestattet, völlig lösemittelfreie Haushaltsalleskleber zu verwenden. Völlig lösemittelfrei heißt hier, daß auch keine Restgehalte an Lösungsmitteln vorliegen. Dies läßt sich dadurch sicherstellen, daß bei der Herstellung der Polyurethandispersionen auf den Einsatz von Lösungsmitteln verzichtet wird. Erfindungsgemäß wird deshalb als Haushaltsalleskleber eine lösemittelfreie Polyurethandispersion bevorzugt eingesetzt, bei deren Herstellung keine Lösungsmittel verwendet werden.

Die den erfindungsgemäß eingesetzten Polyurethan-Dispersionen zugrundeliegenden Polypropylenglykole können weiterhin bis zu 70 Gew.-%, vorzugsweise jedoch weniger, durch andere, in derartigen Zubereitungen übliche Polyole ersetzt sein. Ganz allgemein gilt hier, daß diese anderen Polyole über zumindest zwei reaktionsfähige Wasserstoffatome verfügen müssen und im wesentlichen linear sind, wobei das Molekulargewicht zwischen 300 und 20.000, vorzugsweise zwischen 400 und 6.000 liegen kann. Geeignet sind hier beispielsweise Polyester, Polyacetale, Polycarbonate, Polyether, Polythioether, Polyamide und/oder Polyesteramide, die jeweils im Mittel 2 bis höchstens 4 Hydroxylgruppen aufweisen.

Unter Polycarbonate werden hier Polyester verstanden, die theoretisch durch Veresterung der Kohlensäure mit zwei- oder mehrwertigen Alkoholen dargestellt werden können, und an beiden Kettenenden jeweils eine Hydroxylgruppe aufweisen. Vorzugsweise besitzen die Alkohole und letztlich damit auch die Polycarbonatdiole einen aliphatischen Aufbau. Geeignete mehrwertige Alkohole können z.B. dreiwertig sein wie etwa das Glycerin. Bevorzugt werden jedoch zweiwertige Alkohole, insbesondere wenn diese nicht weniger als vier und nicht mehr als zehn C-Atome aufweisen. Cyclische und verzweigtkettige Alkohole sind zwar geeignet, jedoch werden lineare bevorzugt. Die Hydroxylgruppen können benachbart, z.B. in 1,2-Stellung, oder auch isoliert angeordnet sein. Bevorzugt werden Diole mit terminalen OH-Gruppen. Geeignete Polycarbonatdiole haben ein Molekulargewicht von 500 bis 8000, insbesondere von 800 bis 2500.

Als Polyether seien z.B. die Polymerisationsprodukte des Ethylenoxids, Butylenoxids sowie ihre Misch- oder Pfropfpolymerisationsprodukte sowie die durch Kondensation von mehrwertigen Alkoholen oder Mischungen derselben und die durch Alkoxylierung von mehrwertigen Alkoholen, Aminen, Polyaminen und Aminoalkoholen gewonnenen Polyether genannt. Als Polyether sind auch die in der genannten EP 354 471 beschriebenen Polytetrahydrofurane als auch Ethylenglykol-endständige Polypropylenglykole geeignet.

Als Polyacetale kommen z. B. die aus Glykolen wie Diethylenglykol, Triethylenglykol, Hexandiol und Formaldehyd herstellbaren Verbindungen in Frage. Auch durch Polymerisation cyclischer Acetale lassen sich geeignete Polyacetale herstellen.

Unter den Polythioethern seien insbesondere die Kondensationsprodukte von Thiodiglykol mit sich selbst und/oder mit anderen Glykolen, Dicarbonsäuren, Formaldehyd, Aminocarbonsäuren oder Aminoalkoholen angeführt. Je nach den Co-Komponenten handelt es sich bei den Produkten um Polythioether, Polythiomischether, Polythioetherester, Polythioetheresteramide Derartige Polyhydroxylverbindungen können auch in alkylierter Form bzw. in Mischung mit Alkylierungsmitteln angewandt werden.

Zu den Polyestern, Polyesteramiden und Polyamiden zählen die aus mehrwertigen gesättigten und ungesättigten Carbonsäuren bzw. deren Anhydriden und mehrwertigen gesättigten und ungesättigten Alkoholen, Aminoalkoholen, Diaminen, Polyaminen und ihren Mischungen gewonnenen, überwiegend linearen Kondensate, z.B. Polyterephthalate. Auch Polyester aus Lactonen, z. B. Caprolacton oder aus Hydroxycarbonsäuren sind verwendbar. Die Polyester können Hydroxyloder Carboxylendgruppen aufweisen. Zu ihrem Aufbau können als Alkoholkomponente auch höhermolekulare Polymerisate oder Kondensate, wie z.B. Polyether, Polyacetale, Polyoxymethylene (mit)verwendet werden.

Auch bereits Urethan- oder Harnstoffgruppen enthaltende Polyhydroxylverbindungen sowie gegebenenfalls modifizierte natürliche Polyole wie Rizinusöl sind verwendbar. Grundsätzlich kommen auch Polyhydroxylverbindungen, welche basische Stickstoffatome aufweisen, in Frage, z. B. polyalkoxylierte primäre Amine oder Polyester bzw. Polythioether, welche Alkyl-diethanolamin einkondensiert enthalten. Weiterhin eingesetzt werden können Polyole, die durch vollständige oder teilweise Ringöffnung epoxidierter Triglyceride mit primären oder sekundären Hydroxylverbindungen erzeugt werden können, beispielsweise das Umsetzungsprodukt von epoxidiertem Sojaöl mit Methanol. Auch Mischpolymerisate der genannten Polyhydroxylverbindungen können geeignet sein, ebenso wie deren Analoge mit vorzugsweise endständigen Amino- oder Sulfidgruppen.

Erfindungsgemäß wird bevorzugt, daß bei teilweise aus Polypropylenglykol bestehenden Polyolmischungen der Rest zu 100 Gew.-% der Mischung aus Polytetrahydrofuranen, Polyethylenglykolen, Polyacetalen, Polycarbonaten und/oder Polyestern mit jeweils im Mittel 2 bis höchstens 4 OH-Gruppen besteht.

Die den Polyurethan-Dispersionen zugrundeliegende Mischung mehrfunktioneller Isocyanate besteht ganz oder teilweise aus alpha-alpha-alpha'-alpha'-Tetramethylxyloldiisocyanat (TMXDI). Die meta- und/oder para-isomere Form ist besonders geeignet. Nur mit einem Mindestanteil an TMXDI in der Isocyanatmischung erhält man mit einer auf Polypropylenglykol basierenden Polyolkomponente eine Polyurethandispersion, die als Haushaltsalleskleber verwendet, zu guten Haftungswerten führt. Deshalb besteht die Isocyanatmischung bevorzugt zu mindestens 20 Gew.-%, besser noch zu mindestens 35 Gew.-%, aus TMXDI. Als Faustregel kann gelten, daß die genannten Haftungswerte umso besser ausfallen, je höher der Anteil an TMXDI in der Isocyanatmischung ist. Es werden daher Mischungen bevorzugt, die zur Hälfte oder mehr, etwa zu zwei Dritteln oder drei Vierteln, TMXDI enthalten.

Als zusätzliche, also als Rest zu 100 Gew.-%, Polyisocyanate sind alle mehrfunktionellen, aromatischen und aliphatischen Isocyanate geeignet, wie z. B. 1,5-Naphthylendiisocyanat, 4,4'-Diphenylmethandiisocyanat, 4,4'-Diphenyldimethylmethandiisocyanat, Di- und Tetraalkyldiphenylmethandiisocyanat, 4,4'-Dibenzyldiisocyanat, 1,3-Phenylendiisocyanat, 1,4-Phenylendiisocyanat, die Isomeren des Toluylendiisocyanats, gegebenenfalls in Mischung, 1-Methyl-2,4-diisocyanato-cyclohexan, 1,6-Diisocyanato-2,2,4-trimethylhexan, 1,6-Diisocyanato-2,4,4-trimethylhexan, 1-Isocyanatomethyl-3-isocyanato-1,5,5-trimethyl-cyclohexan, chlorierte und bromierte Diisocyanate, phosphorhaltige Diisocyanate, 4,4'-Diisocyanatophenylperfluorethan, Tetramethoxybutan-1,4-diisocyanat, Butan-1,4-diisocyanat, Hexan-1,6-diisocyanat, Dicyclohexylmethandiisocyanat, Cyclohexan-1,4-diisocyanat, Ethylen-diisocyanat, Phthalsäure-bis-isocyanatoethylester, ferner Polyisocyanate mit reaktionsfähigen Halogenatomen, wie 1-Chlormethylphenyl-2,4-diisocyanat, 1-Brommethylphenyl-2,6-diisocyanat, 3,3-Bis-chlormethylether-4,4'-diphenyldiisocyanat. Schwefelhaltige Polyisocyanate erhält man beispielsweise durch Umsetzung von 2 mol Hexamethylen-diisocyanat mit 1 mol Thiodiglykol oder Dihydroxydihexylsulfid. Weitere wichtige Diisocyanate sind Trimethylhexamethylendiisocyanat, 1,4-Diisocyanatobutan, 1,2-Diisocyanatododecan und Dimerfettsäure-diisocyanat. Interesse verdienen teilweise verkappte Polyisocyanate, welche die Bildung selbstvernetzender Polyurethane ermöglichen, z.B. dimeres Toluylendiisocyanat, oder mit beispielsweise Phenolen, tertiärem Butanol, Phthalimid, Caprolactam partiell umgesetzte Polyisocyanate.

Bei einer bevorzugten Ausführungsform enthält die Isocyanatkomponente zumindest anteilsweise Dimerfettsäureisocyanat. Als Dimerfettsäure bezeichnet man ein Gemisch von überwiegend C ₃₆-Dicarbonsäuren, das durch thermische oder katalytische Dimerisierung ungesättigter C₁₈-Monocarbonsäuren, wie Ölsäure, Tallölfettsäure oder Linolsäure, hergestellt wird. Derartige Dimerfettsäuren sind dem Fachmann seit langem bekannt und kommerziell erhältlich. Die Dimerfettsäure läßt sich zu Dimerfettsäureisocyanaten umsetzen. Im Sinne der Erfindung wird Dimerfettsäurediisocyanat bevorzugt. Technisches Dimerfettsäurediisocyanat besitzt im Mittel mindestens zwei und weniger als drei Isocyanatgruppen pro Molekül Dimerfettsäure.

Die vorgenannten Isocyanate können allein oder auch in Mischung eingesetzt werden. Bevorzugt sind - insbesondere cyclische oder verzweigte - aliphatische Diisocyanate, insbesondere Isophorondiisocyanat.

Vorzugsweise enthalten die geeigneten mehrfunktionellen Isocyanate im Mittel 2 bis höchstens 4 NCO-Gruppen. Die Mengen an der Polyolmischung und an der Mischung mehrfunktioneller Isocyanate werden so gewählt, daß ein bestimmtes Verhältnis von NCO-reaktiven Gruppen zu NCO-Gruppen vorliegt. Bevorzugterweise liegt die Isocyanatkomponente stöchiometrisch nicht im Unterschuß vor, übersteigt andererseits aber auch nicht das Zweieinhalbfache an NCO-reaktiven Gruppen. Besonders günstig ist ein Verhältnis von 1 : 1,05 bis 1 : 2. Der bevorzugte, hinsichtlich der späteren anwendungstechnischen Ergebnisse optimale Bereich liegt zwischen 1 : 1,1 und 1 : 1,6.

Zu den Kettenverlängerungsmitteln mit reaktionsfähigen Wasserstoffatomen zählen:
- die üblichen gesättigten und ungesättigten Glykole, wie Ethylenglykol oder Kondensate des Ethylenglykols, Butandiol-1,3, Butandiol-1,4, Butendiol, Propandiol-1,2, Propandiol-1,3, Neopentylglykol, Hexandiol, Bis-hydroxymethyl-cyclohexan, Dioxyethoxyhydrochinon, Terephthalsäure-bis-glykolester, Bernsteinsäure-di-2-hydroxyethylamid, Bernsteinsäure-di-N-methyl-(2-hydroxy-ethyl)amid, 1,4-Di(2-hydroxy-methyl-mercapto)-2,3,5,6-tetrachlorbenzol, 2-Methylenpropandiol-(1,3), 2-Methyl-propandiol-(1,3);
- aliphatische, cycloaliphatische und aromatische Diamine wie Ethylendiamin, Hexamethylendiamin, 1,4-Cyclohexylendiamin, Piperazin, N-Methyl-propylendiamin, Diaminodiphenylsulfon, Diaminodiphenylether, Diaminodiphenyldimethylmethan, 2,4-Diamino-6-phenyltriazin, Isophorondiamin, Dimerfettsäurediamin;
   Auszuschließen sind Diamine mit nicht gewünschten Stoffeigenschaften, die die Gesundheit beeinträchtigen könnten, so beispielsweise Hydrazin, Diaminodiphenylmethan oder die Isomeren des Phenylendiamins. Weiterhin auch Carbohydrazide oder Hydrazide von Dicarbonsäuren.

- Aminoalkohole wie Ethanolamin, Propanolamin, Butanolamin, N-Methyl-ethanolamin, N-Methyl-isopropanolamin;
- aliphatische, cycloaliphatische, aromatische und heterocyclische Mono- und Diaminocarbonsäuren wie Glycin, 1- und 2-Alanin, 6-Aminocapronsäure, 4-Aminobuttersäure, die isomeren Mono- und Diaminobenzoesäuren, die isomeren Mono- und Diaminonaphthoesäuren;
- Wasser
Es ist hervorzuheben, daß im Rahmen vorliegender Erfindung nicht streng zwischen den Verbindungen mit reaktionsfähigen Wasserstoffatomen mit einem Molekulargewicht von 300 bis 20 000 und den sogenannten "Kettenverlängerungsmitteln" unterschieden werden kann, da die Übergänge zwischen den beiden Verbindungsklassen fließend sind. Verbindungen, die nicht aus mehreren Monomereinheiten aufgebaut sind, jedoch ein Molekulargewicht über 300 aufweisen, wie z.B. 3,3'-Dibrom-4,4'-diaminodiphenylmethan, werden zu den Kettenverlängerungsmitteln gerechnet, ebenso jedoch Pentaethylenglykol, obwohl letzteres seiner Zusammensetzung nach eigentlich ein Polyetherdiol ist.

Spezielle Kettenverlängerungsmittel mit mindestens einem basischen Stickstoffatom sind z.B. mono-, bis- oder polyoxalkylierte aliphatische, cycloaliphatische, aromatische oder heterocyclische primäre Amine, wie N-Methyldiethanolamin, N-Ethyl-diethanolamin, N-Propyl-diethanolamin, N-Isopropyl-diethanolamin, N-Butyl-diethanolamin, N-Isobutyl-diethanolamin, N-Oleyl-diethanolamin, N-Stearyl-diethanolamin, oxethyliertes Kokosfettamin, N-Allyl-diethanolamin, N-Methyl-diisopropanolamin, N-Ethyl-diisopropanolamin, N-Propyl-diisopropanolamin, N-Butyl-diisopropanolamin, C-Cyclohexyl-diisopropanolamin, N,N-Dioxethylanilin, N,N-Dioxethyltoluidin, N,N-Dioxethyl-1-aminopyridin, N,N'-Dioxethyl-piperazin, Dimethyl-bis-oxethylhydrazin, N,N'-Bis-(2-hydroxy-ethyl)-N,N'-diethyl-hexahydro-p-phenylendiamin, N-12-Hydroxyethyl-piperazin, polyalkoxylierte Amine wie oxypropyliertes Methyl-diethanolamin, ferner Verbindungen wie N-Methyl-N,N-bis-3-aminopropylamin, N-(3-Aminopropyl)-N,N'-dimethylethylendiamin, N-(3-Aminopropyl)-N-methyl-ethanolamin, N,N'-Bis-(3-aminopropyl)-N,N'-dimethylethylendiamin, N, N'-Bis(3-aminopropyl)-piperazin, N-(2-Aminoethyl)-piperazin, N, N'-Bisoxyethyl-propylendiamin, 2,6-Diaminopyridin, Diethanolamino-acetamid, Diethanolamidopropionamid, N,N-Bis-oxyethyl-phenyl-thiosemicarbazid, N,N-Bis-oxethyl-methyl-semicarbazid, p, p'-Bis-aminomethyl-dibenzylmethylamin, 2,6-Diaminopyridin, 2-Dimethylaminomethyl-2-methyl-propandiol-1,3.

Kettenverlängerungsmittel mit zur Quaternierung befähigten Halogenatomen bzw. R-SO₂O-Gruppen sind beispielsweise Glycerin-1-chlorhydrin, Glycerinmonotosylat, Pentaerythrit-bis-benzolsulfonat, Glycerin-monomethansulfonat, Addukte aus Diethanolamin und chlormethylierten aromatischen Isocyanaten oder aliphatischen Halogenisocyanaten wie N,N-Bis-hydroxyethyl-N'-m-chlormethylphenylharnstoff, N-Hydroxyethyl-N'-chlorhexylharnstoff, Glycerin-mono-chlorethyl-urethan, Bromacetyl-dipropylentriamin, Chloressigsäurediethanolamid. Als Kettenverlängerungsmittel bevorzugt werden kurzkettige gegenüber Isocyanaten reaktive Diamine und/oder Dihydroxy-Verbindungen.

Die den erfindungsgemäß eingesetzten Polyurethan-Dispersionen zugrundeliegenden Polyurethane enthalten desweiteren als in wäßriger Lösung zur Salzbildung befähigte funktionelle Komponenten Carbonsäuren, Sulfonsäuren oder Ammoniumverbindungen mit 1 bis 2 gegenüber Isocyanaten reaktiven Gruppen in einkondensierter Form. Als solche können eingesetzt werden Dihydroxy- oder auch Diamino-Verbindungen, die eine ionisierbare Carbonsäure-, Sulfonsäure- oder Ammoniumgruppe enthalten. Diese Verbindungen können entweder als solche eingesetzt werden oder sie können in-situ hergestellt werden. Um ionisierbare Carbonsäuregruppen tragende Verbindungen in das Polyurethan einzubringen, kann der Fachmann den Polyolen speziellen Dihydroxycarbonsäuren zugeben, die nicht oder nur im untergeordneten Maß zu Nebenreaktionen der Carboxylgruppen mit den Isocyanatgruppen befähigt sind. Eine bevorzugte Dihydroxycarbonsäure ist beispielsweise die Dimethylolpropionsäure.

Um zur Salzbildung befähigte Sulfonsäuregruppen einzuführen, kann den Polyolen eine Diaminosulfonsäure zugesetzt werden. Beispiele sind 2,4-Diaminobenzolsulfonsäure aber auch die N-(omega-Aminoalkan)-omega'-aminoalkansulfonsäuren wie sie in der DE 20 35 732 beschrieben sind.

Um zur Salzbildung befähigte Ammoniumgruppen in das Polymere einzufügen, kann auch gemäß der bereits zitierten DE 15 95 602 das Polyurethan-Prepolymere mit Hilfe eines aliphatischen und aromatischen Diamins so modifiziert werden, daß an den Kettenenden primäre Aminogruppen vorliegen, die dann mit üblichen Alkylierungsmitteln in quartäre Ammoniumverbindungen oder in Aminsalze überführt werden können.

Erfindungsgemäß ist es bevorzugt, die zu verwendenden Polyurethan-Prepolymeren mit Hilfe von Carbonsäure- oder Sulfonsäuregruppen wasserlöslich bzw. redispergierbar zu machen, da Polyurethan-Dispersionen, die derartige anionische Modifizierungsmittel enthalten, unter alkalischen Bedingungen wieder abgelöst werden können, d.h. derartige Klebstoffe sind unter Waschbedingungen aus bestimmten Substraten, z.B. aus Textilien wieder entfernbar.

In den erfindungsgemäß eingesetzten Polyurethan-Dispersionen liegen die Polymeren in Salzform vor. Bei den bevorzugten mit Carbonsäuren oder Sulfonsäuren modifizierten Polymeren liegen als Gegenionen Alkalimetallsalze, Ammoniak oder Amine, d.h. primäre, sekundäre oder tertiäre Amine vor. Bei den kationisch modifizierten Produkten liegen als Gegenionen Säureanionen, z. B. Chlorid, Sulfat oder die Anionen organischer Carbonsäuren vor. Die zur Salzbildung befähigten Gruppen können daher durch die Gegenionen teil- oder vollständig neutralisiert werden. Auch ein Überschuß an Neutralisationsmittel ist möglich.

Um im wesentlichen klare, d. h. opake bis wasserklare Polyurethan-Dispersionen zu erhalten, muß der Fachmann auf ein bestimmtes Verhältnis zwischen der zur Salzbildung befähigten Komponente und den übrigen, das Polyurethan aufbauenden Stoffe achten. So ist es zweckmäßig, die zur Salzbildung befähigte Komponente in Mengen von 10 bis 90 Mol-%, vorzugsweise von 20 bis 80 Mol-%, und insbesondere von 30 bis 70 Mol-%, bezogen auf Polyolmischung plus dem gewünschtenfalls enthaltenen Kettenverlängerungsmittel, einzusetzen. Weiterhin hängt die Transparenz vom Neutralisationsgrad ab. Dabei kann der Fachmann durch wenige Vorversuche feststellen, ab welcher Menge an Ionenbildung befähigtem Modifizierungsmittel bzw. ab welcher Menge an Neutralisationsmittel ein ausreichender Grad an Transparenz erreicht ist. Im allgemeinen wird so wenig wie möglich von diesen Stoffen eingesetzt werden, da sie bei überhöhtem Einsatz die Wasserfestigkeit bzw. die Feuchtklimafestigkeit des Klebefilms stören können.

Unter den erfindungsgemäßen Polyurethan-Dispersionen werden zweiphasige Wasser/Polyurethan-Systeme verstanden, welche bevorzugt kolloidale Systeme und Sole mit Teilchendurchmessern bis etwa 200 nm umfassen. Diese Systeme sind vorzugsweise optisch opak bis transparent.

Der Feststoffgehalt der erfindungsgemäßen Klebstofflösungen kann in weiten Grenzen variiert werden. In der Praxis haben sich Feststoffgehalte zwischen 20 und 70 Gew.-%, insbesondere zwischen 30 und 50 Gew.-% bewährt.

Wie schon bei der Diskussion der erfindungsgemäß verwendbaren Kettenverlängerungsmittel ausgeführt, sind Verbindungen mit gesundheitsschädlichen oder -beeinträchtigenden Eigenschaften nicht erwünscht. Dies gilt um so mehr, als es sich bei der vorliegenden Erfindung um einen universell einsetzbaren Alleskleber handelt, der vermutlich auch von gesundheitlich stärker gefährdeten Personenkreisen, wie Kindern, Alten, Kranken und Schwangeren verwendet werden wird. Deshalb ist bei einer besonders bevorzugten Ausführungsform nicht nur bei den Kettenverlängerungsmitteln auf physiologisch bedenkliche Stoffe wie Hydrazin zu verzichten, sondern auch bei den übrigen Komponenten des Klebstoffs auf eine möglichst weitgehende physiologische Unbedenklichkeit zu achten. Die im Zusammenhang mit Polyurethanklebstoffen oftmals diskutierte Gesundheitsgefährdung durch freie Isocyanate bzw. nicht abreagierte NCO-Gruppen der Polymeren oder Prepolymeren ist bei der vorliegenden Erfindung gegenstandslos, da hier die Polyurethane in Wasser dispergiert sind und Isocyanatgruppen, wie dem Fachmann bekannt ist, mit Wasser abreagieren, so daß als sichergestellt gelten kann, daß die vorliegenden erfindungsgemäßen Polyurethan-Dispersionen keine reaktiven NCO-Gruppen aufweisen.

Zur Herstellung der für die erfindungsgemäßen Zwecke insbesondere geeigneten Polyurethane werden die Polyole und ein Überschuß an Diisocyanat unter Bildung eines Polymers mit endständigen Isocyanatgruppen umgesetzt, wobei geeignete Reaktionsbedingungen und Reaktionszeiten sowie Temperaturen je nach dem betreffenden Isocyanat variiert werden können. Der Fachmann weiß, daß die Reaktionsfähigkeit der umzusetzenden Bestandteile ein entsprechendes Gleichgewicht zwischen Reaktionsgeschwindigkeit und unerwünschten Nebenreaktionen, die zu einer Verfärbung und ungewollten Molekulargewichtsveränderung führen, notwendig macht. Typischerweise wird die Reaktion unter Rühren bei ungefähr 50 bis ungefähr 120°C innerhalb von ungefähr 1 bis 6 Stunden durchgeführt.

Geeignete Herstellverfahren für Polyurethan-Dispersionen sind z.B. in D. Dieterich, Angew. Makromol. Chem. 98, S. 133 (1981)), Ullmann, Encyklopädie der technischen Chemie, 4. Auflage, Band 19, Verlag Chemie, Weinheim/Bergstraße 1974, S. 311-313, Houben-Weyl, Methoden der organischen Chemie, Band E 20/Teil 1-3, S. 1659-1663 und S. 1671-1681 sowie in Journal of Waterborne Coating, August 1984, S. 2 ff. beschrieben. Aus den in diesen Artikeln genannten Sekundärliteraturstellen kann sich der geneigte Fachmann auch die entsprechende Patentliteratur erschließen. Die bislang als Haushaltsalleskleber geeigneten Polyurethandispersionen werden vorzugsweise, wie aus der bereits genannten EP 354 471 bekannt, nach dem sogenannten Aceton-Verfahren hergestellt. Lösemittelzusätze von Niedrigsiedern wie z.B. Aceton sind hier u.a. nötig, um die Viskosität des Prepolymeren zu senken und dieses damit handhabbar zu machen, wodurch die Dispergierung erst möglich wird. Der Nachteil solcher Herstellverfahren ist unter der Berücksichtigung der Forderung nach lösemittelfreien Allesklebern, das ein technisch aufwendiger Destillationsschritt an das Dispergierverfahren angeschlossen werden muß, um den Niedrigsieder zumindestens überwiegend zu entfernen. Dieses bedeutet einen zusätzlichen Verfahrensschritt, der das Verfahren nicht nur verkompliziert sondern auch zu einer Verteuerung des Produkts führt. Dies nicht zuletzt deshalb, weil das vorzugsweise eingesetzte Aceton nicht wieder ohne weiteres in den Prozeß rückgeführt werden kann, da vorzugsweise wasserfreies Aceton eingesetzt wird. Damit ist für den Fachmann u.a. auch die Frage verknüpft, ob und gegebenenfalls bis zu welcher Menge, ein Restlösemittelgehalt akzeptabel ist, da davon der Verfahrensaufwand abhängt. Dem steht allerdings die Forderung nach einem lösungsmittelfreien Produkt entgegen. Diese Forderung ergibt sich gerade im konsumnahen Bereich durch die öffentliche Diskussion um Restmengen an Lösungsmitteln in z.B. Haushaltsprodukten. Es besteht daher ein Bedarf an Verfahren, die einerseits zu den gewünschten Produkten führen, ohne deren gewünschte Eigenschaften zu beeinträchtigen und andererseits völlig ohne Lösungsmittel durchgeführt werden können, da dadurch auch Restgehalte an Lösungsmitteln vermieden werden können.

Die bislang bekannten, als Haushaltsalleskleber geeigneten Polyurethan-Dispersionen sind nicht nach einer Variante des lösungsmittelfreien Dispergierverfahrens herstellbar, da schon die Vorprodukte eine zu hohe Viskosität aufweisen. Andererseits sind bislang bekannte, nach lösungsmittelfreien Dispergierverfahren herstellbare Polyurethandispersionen nicht als Haushaltsalleskleber geeignet. Derartige Polyurethandispersionen sind z.B. solche, die als wesentlichen Bestandteil der Polyolkomponente Polypropylenglykol enthalten. Wie im Vergleichsbeispiel 1 des Beispielteils dieser Anmeldung, welches dem Vergleichsbeispiel 1 aus der genannten EP 354 471 entspricht, zu erkennen ist, weisen derartige Polyurethandispersionen eine zu niedrige Haftung an Substratoberflächen insbesondere Kunststoffoberflächen auf, die sie als Haushaltsalleskleber ungeeignet machen. Generell ist dem Fachmann diesbezüglich wohlbekannt, daß Polyurethanklebstoffe auf Basis von Polypropylenglykol deutlich schlechtere Haftwerte bzw. Zugscherfestigkeiten an nahezu allen Oberflächen besitzen, als beispielsweise Polyurethanklebstoffe auf Basis von Polyesterpolyolen.

Erfindungsgemäß wurde gefunden, daß die Herstellung einer als Haushaltsalleskleber geeigneten im wesentlichen klaren und zumindest weitgehend lösemittelfreien wäßrigen einkomponentigen Polyurethan-Dispersion auf Basis der Reaktionsprodukte
- einer ganz oder teilweise aus Polypropylenglykol bestehenden Polyolmischung,
- einer ganz oder teilweise aus alpha-alpha-alpha'-alpha'-Tetramethylxyloldiisocyanat (TMXDI) bestehenden Mischung mehrfunktioneller Isocyanate,
- einer in wäßriger Lösung zur Salzbildung befähigten funktionellen Komponente und
- gewünschtenfalls eines Kettenverlängerungsmittels,
nach einem lösungsmittelfreien Verfahren hergestellt werden kann. Mit anderen Worten, die Umsetzung der genannten Reaktanten zu den Reaktionsprodukten sowie die Herstellung der wäßrigen Dispersion der aus dieser Umsetzung resultierenden Prepolymerphase kann in Abwesenheit inerter Lösungsmittel durchgeführt werden. Dabei wird üblicherweise so verfahren, daß die vorstehend beschriebenen Reaktanten bei Raumtemperatur gemischt werden. Im allgemeinen kann die Reaktion in üblichen Kesselanlagen durchgeführt werden. Die Temperatur bei Reaktionsdurchführung liegt etwa zwischen 70 und 110°C. Die Reaktionsmischung kann die für Polyurethanreaktionen wirksame Katalysatorzusätze enthalten. Es wird üblicherweise so lange gerührt, bis sich der gewünschte NCO-Wert einstellt. Die Reaktion kann nach dem sogenannten 1- aber auch nach dem sogenannten 2-Reaktor-Verfahren durchgeführt werden. Im ersten Fall wird unter Einleitung von Wasser mit der zur Neutralisation gewünschten Basenmenge unter kräftigem Rühren die Polyurethanprepolymeren dispergiert. Andererseits kann aber auch die Prepolymerphase in die wäßrige Basenlösung eingleitet und dort unter kräftigem Rühren dispergiert werden. In beiden Fällen kann die Dispergierung bei erhöhten Temperaturen stattfinden, an die sich gegebenenfalls noch eine ein- bis zweistündige Nachrührphase anschließt.

Die vorstehende Erfindung wird durch nachstehende Beispiele näher erläutert.

### Beispiele

### Allgemeine Herstellvorschriften

Die Reaktionskomponenten wurden bei Raumtemperatur gemischt und in einer üblichen Kesselanlage bei Temperaturen zwischen 70 und 110°C gerührt, bis der NCO-Gehalt nicht mehr sinkt. Unter Einleitung mit der zur Neutralisation gewünschten Basenmenge und unter kräftigem Rühren erfolgte die Dispergierung. Anschließend wurde noch 1 bis 2 Stunden nachgerührt.

### Beispiel 1:

0,5 Mol Dimethylolpropionsäure
0,1 Mol Polypropylenglykol MG 400
0,1 Mol Polytetrahydrofuran MG 650
0,3 Mol Polypropylenglykol MG 1000
1,4 Mol TMXDI
Festkörpergehalt: 35 %, Viskosität: 3.500 mPas
Aussehen: opak-transparent

### Beispiel 2:

0,55 Mol Dimethylolpropionsäure
0,15 Mol Polypropylenglykol MG 400
0,25 Mol Polypropylenglykol MG 1000
0,05 Mol Polypropylenglykol MG 2000
0,44 Mol IPDI
0,86 Mol TMXDI
Festkörpergehalt: 34,2 %, Viskosität: 3.000 mPas
Aussehen: opak-transparent

### Vergleichsbeispiel 1 und 2 hergestellt nach dem Acetonverfahren:

Die Polyole werden mit Aceton verdünnt oder darin gelöst bzw. dispergiert. Unter Rühren erfolgt dann die Zugabe der zur Ionenbildung befähigten Komponente. Bei Temperaturen zwischen 50 und 70°C wird dann Diisocyanat so lange zugegeben, bis der NCO-Gehalt nicht mehr sinkt. Es erfolgt dann die Zugabe von Wasser, das die zur Neutralisation benötigten Basen enthält. Flüssige tertiäre Amine können eventuell auch kurz vor dem Dispergieren dem Prepolymeren hinzugefügt werden. Nach mindestens 30-minütigem Dispergieren wird dann das Aceton abdestilliert, später unter höherem Vakuum bei Temperaturen von 55 bis 60°C.

### Vergleichsbeispiel 1

| | | |
|---|---|---|
| Polypropylenglykol, gestartet auf | 100 GT | 0,09 Mol |
| Glycerin, OHZ 34, MG 3500 | | |
| oleochemisches Polyol, entspr. | 82 GT | 0,36 Mol |
| DE-PS 37 04 350, OHZ 160 | | |
| Dimethylolpropionsäure | 21,50 GT | 0,55 Mol |
| Aceton | 50 GT | 2,0 Mol |
| Isophorondiisocyanat | 98,05 GT | 1,45 Mol |
| Natriumhydroxyd | 6,40 GT | 0,36 Mol |
| Wasser, deionisiert | 450 GT | 56,0 Mol |

### Vergleichsbeispiel 2

0,50 Mol Dimethylolpropionsäure
0,20 Mol Polypropylenglykol MG 400
0,30 Mol Polypropylenglykol MG 1000
0,6 Mol Isophorondiisocyanat
0,7 Mol Hexandiisocyanat
Festkörpergehalt: 34 %, Viskosität: 4.000 mPas
Aussehen: opak-transparent

| Klebkraft (Zugscherfestigkeit, Anlehnung an "DIN 53254") in N/mm² | | | | |
|---|---|---|---|---|
| | Beispiel 1 | Beispiel 2 | Vergleichs-Beispiel 1 | Vergleichs-Beispiel 2 |
| Holz*/Holz* | 8,4 | 7,8 | 4,5 | 7,0 |
| Holz*/ABS | 3,5 | 3,3 | 1,4 | 1,8 |
| Holz*/Plexiglas | 5,6 | 5,3 | 1,5 | 2,0 |
| Holz*/Alu | 7,5 | 7,6 | 4,9 | 6,5 |
| Die Prüfkörper (5 x 10 cm²) wurden nach leichtem Anrauhen verklebt und nach 3 Tagen mit einer Vorschubgeschwindigkeit von 10 cm/Min. zerrissen. | | | | |

| | | | | |
|---|---|---|---|---|
| * Buchensperrholz MG in obigen Beispielen bedeutet: mittleres Molekulargewicht (ca. Werte). | | | | |

## Patentansprüche

1. Verwendung einer opaken bis wasserklaren und zumindest weitgehend lösemittelfreien wäßrigen einkomponentigen Polyurethan-Dispersion auf Basis der Reaktionsprodukte
- einer ganz oder teilweise aus Polypropylenglykol bestehenden Polyolmischung,
- einer ganz oder teilweise aus alpha-alpha-alpha'-alpha'-Tetramethylxyloldiisocyanat (TMXDI) bestehenden Mischung mehrfunktioneller Isocyanate,
- einer in wäßriger Lösung zur Salzbildung befähigten funktionellen Komponente und
- gewünschtenfalls eines Kettenverlängerungsmittels
als Haushaltsalleskleber.

2. Ausführungsform nach Anspruch 1, dadurch gekennzeichnet, daß das in den Polyolmischungen enthaltene Polypropylenglykol einen Molekulargewichtsbereich von 200 bis 5000 aufweist.

3. Ausführungsform nach einem der vorstehenden Ansprüche, dadurch gekennzeichnet, daß als Haushaltsalleskleber eine lösemittelfreie Polyurethandispersion eingesetzt wird, bei deren Herstellung keine Lösungsmittel verwendet werden.

4. Ausführungsform nach einem der vorstehenden Ansprüche, dadurch gekennzeichnet, daß die den Polyurethan-Dispersionen zugrundeliegenden Polyolmischungen mindestens 30 Gew.-% Polypropylenglykol enthalten.

5. Ausführungsform nach einem der vorstehenden Ansprüche, dadurch gekennzeichnet, daß bei teilweise aus Polypropylenglykol bestehenden Polyolmischungen der Rest zu 100 Gew.-% der Mischung aus Polytetrahydrofuranen, Polyethylenglykolen, Polyacetalen, Polycarbonaten und/oder Polyestern mit jeweils im Mittel 2 bis höchstens 4 OH-Gruppen besteht.

6. Ausführungsform nach einem der vorstehenden Ansprüche, dadurch gekennzeichnet, daß die Mischung mehrfunktioneller Isocyanate mindestens 20 Gew.-% TMXDI enthält.

7. Ausführungsform nach einem der vorstehenden Ansprüche, dadurch gekennzeichnet, daß bei teilweise aus TMXDI bestehenden Mischungen mehrfunktioneller Isocyanate der Rest zu 100 Gew.-% der Mischung aus Isocyanaten mit im Mittel 2 bis höchstens 4 NCO-Gruppen besteht.

8. Ausführungsform mach einem der vorstehenden Ansprüche, dadurch gekennzeichnet, daß die Mengen der Polyolmischung und der Mischung mehrfunktioneller Isocyanate so gewählt sind, daß ein Verhältnis von NCO-reaktiven Gruppen zu NCO-Gruppen in einem Bereich von 1 : 1 bis 1 : 2,5 vorliegt.

9. Ausführungsform nach einem der vorstehenden Ansprüche, dadurch gekennzeichnet, daß die den Polyurethan-Dispersionen zugrundeliegenden Polyurethane als in wäßriger Lösung zur Salzbildung befähigte Komponenten Carbonsäuren, Sulfonsäuren oder Ammoniumverbindungen mit 1 bis 2 gegenüber Isocyanaten reaktiven Gruppen einkondensiert enthalten.

10. Ausführungsform nach einem der vorstehenden Ansprüche, dadurch gekennzeichnet, daß die den Polyurethan-Dispersionen zugrundeliegenden Polyurethane einkondensierte Carbonsäuren oder Sulfonsäuren enthalten, die als Alkalisalz, Ammoniumsalz oder als Salz primärer, sekundärer oder tertiärer Amine vorliegen.

11. Ausführungsform nach einem der vorstehenden Ansprüche, dadurch gekennzeichnet, daß als einkondensierte Carbonsäure Dimethylolpropionsäure vorliegt.

12. Ausführungsform nach einem der vorstehenden Ansprüche, dadurch gekennzeichnet, daß die in wäßriger Lösung zur Salzbildung befähigten Komponenten bezogen auf Polyolmischung plus dem gewünschtenfalls enthaltenen Kettenverlängerungsmittel in einem Bereich von 10 bis 90 Mol-% vorliegen.

13. Ausführungsform nach einem der vorstehenden Ansprüche, dadurch gekennzeichnet, daß die den Polyurethan-Dispersionen zugrundeliegenden Polyurethane als Kettenverlängerungsmittel gegenüber Isocyanatgruppen reaktive Diamine und/oder Dihydroxy-Verbindungen enthalten.

14. Verfahren zur Herstellung einer als Haushaltskleber geeigneten opaken bis wasserklaren und zumindest weitgehend lösemittelfreien wäßrigen einkomponentigen Polyurethan-Dispersion auf Basis der Reaktionsprodukte
- einer ganz oder teilweise aus Polypropylenglykol bestehenden Polyolmischung,
- einer ganz oder teilweise aus alpha-alpha-alpha'-alpha'-Tetramethylxyloldiisocyanat (TMXDI) bestehenden Mischung mehrfunktioneller Isocyanate,
- einer in wäßriger Lösung zur Salzbildung befähigten funktionellen Komponente und
- gewünschtenfalls eines Kettenverlängerungsmittels,
dadurch gekennzeichnet, daß die Umsetzung zu den Reaktionsprodukten sowie die Herstellung der wäßrigen Dispersion der aus dieser Umsetzung resultierenden Prepolymerphase in Abwesenheit inerter Lösungsmittel durchgeführt wird.

## Claims

1. The use of an opaque to water-clear and at least largely solventless, aqueous, one-component polyurethane dispersion based on the reaction products of
- a polyol mixture consisting completely or partly of polypropylene glycol,
- a mixture of polyfunctional isocyanates consisting completely or partly of α,α,α',α'-tetramethyl xylene diisocyanate (TMXDI),
- a functional component capable of salt formation in aqueous solution and
- optionally a chain-extending agent
as a universal household adhesive.

2. The use claimed in claim 1, characterized in that the polypropylene glycol present in the polyol mixtures has a molecular weight in the range from 200 to 5,000.

3. The use claimed in any of the preceding claims, characterized in that a solventless polyurethane dispersion prepared without the use of solvents is used as a universal household adhesive.

4. The use claimed in any of the preceding claims, characterized in that the polyol mixtures on which the polyurethane dispersions are based contain at least 30% by weight of polypropylene glycol.

5. The use claimed in any of the preceding claims, characterized in that, where the polyol mixtures consist partly of polypropylene glycol, the balance to 100% by weight of the mixture consists of polytetrahydrofurans, polyethylene glycols, polyacetals, polycarbonates and/or polyesters containing on average 2 to at most 4 OH groups.

6. The use claimed in any of the preceding claims, characterized in that the mixture of polyfunctional isocyanates contains at least 20% by weight of TMXDI.

7. The use claimed in any of the preceding claims, characterized in that, where the mixtures of polyfunctional isocyanates consist partly of TMXDI, the balance to 100% by weight of the mixture consists of isocyanates containing on average 2 to at most 4 NCO groups.

8. The use claimed in any of the preceding claims, characterized in that the quantities of polyol mixture and the mixture of polyfunctional isocyanates are selected so that the ratio of NCO-reactive groups to NCO groups is in the range from 1 : 1 to 1 : 2.5.

9. The use claimed in any of the preceding claims, characterized in that the polyurethanes on which the polyurethane dispersions are based contain co-condensed carboxylic acids, sulfonic acids or ammonium compounds containing 1 to 2 isocyanate-reactive groups as the components capable of salt formation in aqueous solution.

10. The use claimed in any of the preceding claims, characterized in that the polyurethanes on which the polyurethane dispersions are based contain co-condensed carboxylic acids or sulfonic acids in the form of an alkali salt, ammonium salt or salt of primary, secondary or tertiary amines.

11. The use claimed in any of the preceding claims, characterized in that dimethylol propionic acid is present as the co-condensed carboxylic acid.

12. The use claimed in any of the preceding claims, characterized in that the components capable of salt formation in aqueous solution are present in a quantity of 10 to 90 mol-%, based on the polyol mixture plus the chain-extending agent optionally present.

13. The use claimed in any of the preceding claims, characterized in that the polyurethanes on which the polyurethane dispersions are based contain isocyanate-reactive diamines and/or dihydroxy compounds as chain-extending agents.

14. A process for the production of an opaque to water-clear and at least substantially solventless aqueous one-component polyurethane dispersion based on the reaction products
- of a polyol mixture consisting completely or partly of polypropylene glycol,
- a mixture of polyfunctional isocyanates consisting completely or partly of α,α,α',α'-tetramethyl xylene diisocyanate (TMXDI),
- a functional component capable of salt formation in aqueous solution and
- optionally a chain-extending agent,
characterized in that the reaction to the reaction products and the preparation of the aqueous dispersion of the prepolymer phase resulting from this reaction is carried out in the absence of inert solvents.

## Revendications

1. Utilisation comme colle universelle à usage ménager d'une dispersion de polyuréthane aqueuse, à un composant, opaque à transparente et au moins largement exempte de solvants, à base des produits de réaction
- d'un mélange de polyols constitué en totalité ou en partie de polypropylèneglycol,
- d'un mélange d'isocyanates polyfonctionnels constitué en totalité ou en partie d'alpha-alpha-alpha'-alpha'-tétraméthylxylènediisocyanate (TMXDI),
- d'un composant fonctionnel apte à la salification en solution aqueuse et
- si besoin est, d'un agent d'allongement de chaînes.

2. Forme de réalisation selon la revendication 1, caractérisée en ce que le polypropylèneglycol contenu dans les mélanges de polyols présente un domaine de poids moléculaire de 200 à 5000.

3. Forme de réalisation selon une des revendications qui précèdent, caractérisée en ce que l'on utilise comme colle universelle à usage ménager une dispersion de polyuréthane exempte de solvant, pour la fabrication de laquelle aucun solvant n'est utilisé.

4. Forme de réalisation selon une des revendications qui précèdent, caractérisée en ce que les mélanges de polyols à la base des dispersions de polyuréthane renferment au moins 30 % en poids de polypropylèneglycol.

5. Forme de réalisation selon une des revendications qui précèdent, caractérisée en ce que dans les mélanges de polyols constitués en partie de polypropylèneglycol, le reste pour obtenir 100 % en poids du mélange se compose de polytétrahydrofuranes, polyéthylèneglycols, polyacétals, polycarbonates et/ou polyesters comportant chacun en moyenne 2 à, au maximum, 4 groupes OH.

6. Forme de réalisation selon une des revendications qui précèdent, caractérisée en ce que le mélange d'isocyanates polyfonctionnels contient au moins 20 % en poids de TMXDI.

7. Forme de réalisation selon une des revendications qui précèdent, caractérisée en ce que dans les mélanges d'isocyanates polyfonctionnels constitués en partie de TMXDI, le reste pour obtenir 100 % en poids du mélange est constitué d'isocyanates comportant en moyenne 2 à, au maximum, 4 groupes NCO.

8. Forme de réalisation selon une des revendications qui précèdent, caractérisée en ce que les quantités du mélange de polyols et du mélange d'isocyanates polyfonctionnels sont sélectionnées de manière telle que le rapport entre les groupes NCO-réactifs et les groupes NCO est compris entre 1:1 et 1:2,5.

9. Forme de réalisation selon une des revendications qui précédent, caractérisée en ce que les polyuréthanes à la base des dispersions de polyuréthanes renferment comme composants susceptibles de salification en milieu aqueux, des acides carboxyliques, des acides sulfoniques ou des composés d'ammonium comportant 1 à 2 groupes réactifs vis-à-vis des isocyanates.

10. Forme de réalisation selon une des revendications qui précèdent, caractérisée en ce que les polyuréthanes à la base des dispersions de polyuréthanes renferment des acides carboxyliques ou des acides sulfoniques incorporés par condensation, qui sont présents comme sel de métal alcalin, sel d'ammonium ou sel d'amine primaire, secondaire ou tertiaire.

11. Forme de réalisation selon une des revendications qui précèdent, caractérisée en ce que de l'acide diméthylolpropionique est présent comme acide carboxylique incorporé par condensation.

12. Forme de réalisation selon une des revendications qui précèdent, caractérisée en ce que les composants en solution aqueuse susceptibles de salification sont présents dans une plage de 10 à 90 moles % par rapport au mélange de polyols plus l'agent d'allongement de chaînes éventuellement contenu.

13. Forme de réalisation selon une des revendications qui précèdent, caractérisée en ce que les polyuréthanes à la base des dispersions de polyuréthanes renferment comme agents d'allongement de chaînes, des diamines et/ou des composés dihydroxyle réactifs vis-à-vis des groupes isocyanate.

14. Procédé de fabrication d'une dispersion de polyuréthane à un composant, aqueuse, opaque à transparente et au moins largement exempte de solvants, appropriée comme colle universelle à usage ménager, à base des produits de réaction
- d'un mélange de polyols constitué en totalité ou en partie de polypropylèneglycol,
- d'un mélange d'isocyanates polyfonctionnels constitué en totalité ou en partie d'alpha-alpha-alpha'-alpha'-tétraméthylxylènediisocyanate (TMXDI),
- d'un composant fonctionnel apte à la salification en solution aqueuse et
- si besoin est, d'un agent d'allongement de chaînes.
caractérisé en ce que la transformation en produits réactionnels ainsi que la préparation de la dispersion aqueuse de la phase de prépolymère résultant de ladite transformation sont opérées en l'absence de solvants inertes.
